# EUROPEAN PATENT SPECIFICATION

(11) **EP 0 625 059 B1**
(45) Date of publication and mention of the grant of the patent: **28.08.1996**
(21) Application number: 93914498.6
(22) Date of filing: 28.01.1993
(51) Int. Cl.: A61M 25/01, A61B 8/12

(54) **GUIDE WIRE FOR A CATHETER WITH POSITION INDICATING MEANS**
KATHETERFUERUNGSDRAHT MIT POSITIONANZEIGER
FIL DE GUIDAGE POUR CATHETER AVEC INDICATEUR DE POSITION

(30) Priority: 30.01.1992 GB 9202031
(43) Date of publication of application: 23.11.1994
(73) Proprietor: INTRAVASCULAR RESEARCH LIMITED, London SW15 2NQ (GB)
(72) Inventor: DICKINSON, Robert, Julian, London SW11 6HU (GB); ROTHMAN, Martin Terry, London SE22 8Sy (GB)
(74) Representative: Burrington, Alan Graham Headford
(86) International application number: GB9300189
(87) International publication number: WO9314803

(56) References cited:
- EP-A- 0 050 606
- EP-A- 0 145 489
- WO-A-91/17710
- US-A- 4 867 173
- US-A- 4 917 094
- US-A- 4 951 686
- US-A- 4 991 588

## Description

The present invention relates to catheters and more particularly to means for enabling the position of the tip of a catheter to be identified when it is inside a patient's body.

### Background to the Invention

There are numerous different types of catheter which are used for a multitude of medical purposes many of which involve the insertion of the catheter into a patient's body via, for example, an artery of the patient.

An example of such use is disclosed in our earlier International Patent Application Nos GB88/00971 (Publication No WO89/04142) and GB90/00830 (Publication No WO90/14601). In those earlier patent applications there is disclosed a catheter which has at its distal tip (distal with respect to the operating surgeon) an ultrasonic transducer array adapted to emit ultrasonic signals and to receive their echoes in order to provide an image of the interior of the human organ in question, e.g an artery which could be inside the patient's heart.

Apart from the system disclosed in these two earlier patent specifications and the associated catheters for use in those systems, there is also the well-known balloon type catheter which is used to carry out a so-called angioplasty. This operation involves the insertion of the balloon catheter into a patient's artery, typically through the patient's thigh, and then feeding the distal end of the catheter into the damaged artery which could be within the patient's heart. Typically the damage can consist of so-called plaque or a loose part of the inside wall of the artery, as it were, hanging down into the artery thus impeding blood flow.

In carrying out an angioplasty operation the surgeon will need to accurately locate the balloon distal tip of the catheter in exactly the right position within the artery in relation to the plaque or damaged wall. Furthermore, it may be necessary for the surgeon to withdraw the tip at least to some extent and then to reposition it in the affected area.

Considerable skill, and therefore room for error, is involved in this accurate positioning of the distal end of the catheter.

The present invention is concerned with providing means whereby the surgeon can more easily locate the distal tip of the catheter in the required position within the patient or reposition that tip in the required position.

It is normal, to provide the proximal end of the catheter (proximal with respect to the surgeon) with some kind of "handle" by which the surgeon can grip the catheter in order to push it into the patient's artery and to feed it so that the distal end arrives at the desired position. It is also known to provide a catheter with some mechanical means for indicating to the surgeon the distance that the catheter has been inserted into the patient. More specifically, it is known to employ a motorised drive to pull back the catheter by a prescribed amount.

The present invention is concerned with providing improved means for indicating to the surgeon the position of the catheter within the patient and in particular the position of the distal end of the cather which distal end carries some operative device, such as a balloon, whereby the surgeon is able to effect the required treatment or operation. The distal end of the catheter could be provided with other devices/arrangements than a balloon, these devices/arrangements being of many known kinds.

There is disclosed in US Patent 4,867,173 a catheter having a central guide wire the tip of which can be made of radio opaque material to facilitate location of the distal end of the guide wire.

There is disclosed in European Patent Application 0050606 a dispensing container for venous catheters which container is provided with an indicator by which as the catheter is inserted into the patient the length that has been inserted can be read off.

The scope of the invention is as set out in claim 1.

### Brief Description of Drawings

How the invention may be carried out will now be described, by way of example only, and with reference to the accompanying drawings in which:
FIG 1 shows a known type of catheter to which the present invention can be applied; and
FIG 2 is an enlarged fragmentary view of part of the catheter shown in FIG 1 incorporating an embodiment of the invention;

### Figure 1

This illustrates a typical known type of catheter which consists of a plastic tube 1 which has a handle 2 at one end and a tip member 3 at the other. A guide wire 4 can be passed through the tube 1. The handle 2 is provided with an axially located aperture 5 through which the guide wire 4 can pass. The handle 2 is also provided with further radially located apertures 6 and 7 through which various treatment devices can be inserted into the catheter tube 1.

### Figure 2

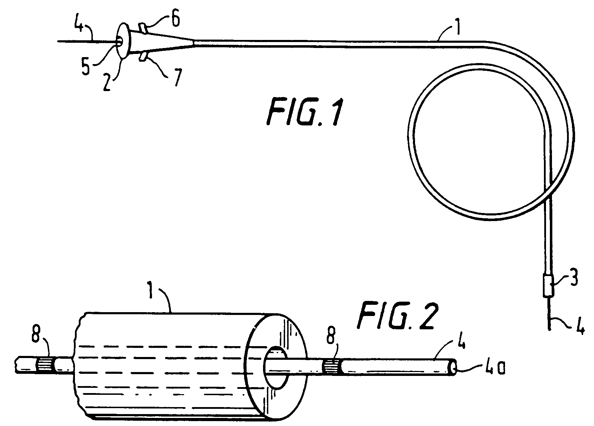

In this embodiment of the present invention parts corresponding to the parts in the known catheter of FIG 1 have the same reference numerals.

The guide wire 4 is provided with a plurality of x-ray-(radio)-opaque markers 8. In a preferred arrangement the markers would be placed 1cm apart along the length of thee guide wire 4 with heavier or duplicate markings at 5cm intervals.

In use the guide wire 4 would be used to position the catheter accurately within the relevant vessel (artery) of the patient using the known x-ray equipment. By providing the markers 8 it is possible to accurately position and reposition the marker wire compared with the prior art catheter without the markers 8.

Another practical difficulty with the prior art is that it is often difficult to estimate the size of the relevant vessel (artery) due to uncertainties concerning the magnification of the visualising system and also pin cushion deformation. By providing markers of known dimensions it is easier for the surgeon to make the aforementioned size estimates.

## Claims

1. A catheter (1) provided with a guide wire (4) over which the catheter can be slid is characterised in that the guide wire is provided with a plurality of X-ray opaque markers (8) set at regular intervals along the length of the wire.

2. A catheter as claimed in claim 1 having two sets of opaque markers, the markers in each set being distinguishable from those in the other set.

3. A catheter as claimed in claim 2 in which the first set of markers are set substantially 1cm apart from one another, and the second set of markers are set substantially 5cm apart from one another.

## Patentansprüche

1. Ein Katheter (1) mit einem Führungsdraht (4) auf den der Katheter aufgeschoben werden kann, dadurch gekennzeichnet, daß der Führungsdraht mehrere undurchsichtige Röntgenmarkierungen aufweist (8), die im regelmäßigen Abstand über der Länge des Drahtes hinweg angeordnet sind.

2. Ein Katheter gemäß Anspruch 1 mit zwei Sätzen undurchsichtiger Markierungen, wobei sich die Markierungen der beiden Sätze von einander unterscheiden lassen.

3. Ein Katheter gemäß Anspruch 2, in dem der erste Satz Markierungen im Abstand von weitaus 1 cm von einander und der zweite Satz Markierungen im Abstand von weitaus 5 cm von einander angeordnet sind.

## Revendications

1. Un cathéter (1) doté d'un fil de guidage (4) sur lequel on peut faire glisser le cathéter est caractérisé en ce que le fil de guidage est doté d'une pluralité de marqueurs opaques radiographiques (8) placés à intervalles réguliers sur toute la longueur du fil.

2. Un cathéter comme revendiqué dans la revendication 1 ayant deux jeux de marqueurs opaques, les marqueurs de chaque jeu étant distinguables de ceux de l'autre jeu.

3. Un cathéter comme revendiqué dans la revendication 2 dans lequel les marqueurs du premier jeu sont placés substantiellement à 1 cm les uns des autres, et les marqueurs du deuxième jeu sont placés substantiellement à 5 cm les uns des autres.
